# EUROPÄISCHE PATENTSCHRIFT

(11) **EP 4 463 096 B1**
(45) Veröffentlichungstag und Bekanntmachung des Hinweises auf die Patenterteilung: **17.12.2025**
(21) Anmeldenummer: 22844540.9
(22) Anmeldetag: 29.12.2022
(51) Int. Cl.: A61B 90/20

(54) **AUTOMATISIERTE REGISTRIERUNG VON PRÄOPERATIVEN VOLUMENBILDDATEN MITTELS SUCHBILD**
AUTOMATED RECORDING OF PRE-SURGERY VOLUME IMAGE DATA, USING A SEARCH IMAGE
ENREGISTREMENT AUTOMATISÉ DE DONNÉES D'IMAGE DE VOLUME DE PRÉ-CHIRURGIE À L'AIDE D'UNE IMAGE DE RECHERCHE

(30) Priorität: 12.01.2022 DE 102022100626
(43) Veröffentlichungstag der Anmeldung: 20.11.2024
(73) Patentinhaber: Carl Zeiss Meditec AG, 07745 Jena (DE)
(72) Erfinder: SAUR, Stefan, 73431 Aalen (DE); HAUGER, Christoph, 73431 Aalen (DE)
(74) Vertreter: Kraus & Lederer PartGmbB
(86) Internationale Anmeldenummer: PCT/EP2022/088028
(87) Internationale Veröffentlichungsnummer: WO 2023/135022

(56) Entgegenhaltungen:
- WO-A1-2021/087433
- US-A- 5 795 294
- US-A1- 2007 270 690
- US-A1- 2015 348 311

## Beschreibung

### TECHNISCHES GEBIET

Verschiedene Beispiele betreffen Techniken, um Messdaten mit Tiefenauflösung für eine Zielregion zu erfassen, wobei mittels der Messdaten mit Tiefenauflösung eine Transformationsvorschrift zwischen mittels eines robotischen Visualisierungssystems erfassten Bilddaten und präoperativen Volumenbilddaten bestimmt werden kann.

### HINTERGRUND

Um beispielsweise für Navigationsanwendungen in der Mikrochirurgie präoperative Volumenbilddaten (z.B. Computer-Tomographie, CT, oder Magnetresonanz-Tomographie, MRT) während der Operation ortsgenau auf die Anatomie des Patienten überlagern zu können, ist eine Registrierung der präoperativen Bilddaten auf die Lage des Patienten im OP notwendig, d.h. es wird eine Transformationsvorschrift bestimmt, die beschreibt, wie präoperative Bilddaten transformiert werden müssen, um ortsgenau auf den Patienten überlagert werden zu können.

Zum Bestimmen der Transformationsvorschrift werden korrespondierende Punkte in den präoperativen Volumenbilddaten und vom Patienten im Operationssaal benötigt. Die Transformationsvorschrift bildet Bilder, die mittels eines robotischen Visualisierungssystems im Operationssaal erfasst werden, auf präoperativen Volumenbilddaten ab, und/oder andersherum.

In manchen Beispielen werden hierzu physische Marker am Patienten befestigt, die sowohl in den präoperativen Volumenbilddaten als auch in Bilddaten eines Visualisierungssystems im Operationssaal sichtbar sind. Bei diesem Ansatz ist es nachteilig, dass die physischen Marker am Patienten befestigt werden müssen, was fehleranfällig und aufwendig ist. Außerdem ist es erforderlich, die physischen Marker sowohl für die Aufnahme der präoperativen Volumenbilddaten, wie auch vor der eigentlichen Operation am Patienten oder ortsfest in Bezug auf den Patienten zu befestigen.

In anderen Beispielen wird ein markerloser Ansatz verfolgt. Dabei ist die Geometrie der Oberfläche (Topographie der Anatomie) einer Zielregion des Patienten sowohl in Messdaten, die im Operationsaal erfasst werden und eine Tiefenauflösung aufweisen, wie auch in den präoperativen Bilddaten sichtbar:
Beispielsweise können mit einem handtragbaren Zeigerinstrument signifikante Punkte der Schädelgeometrie (Ohransatz, Wangen, Nase, ...) berührt und die entsprechenden Messpunkte (die dann im dreidimensionalen Raum definiert sind) der derart gewonnenen Messdaten an das Navigationssystem übertragen werden. Es entsteht eine undichte (engl. "sparse") Abtastung der Oberfläche des Schädels, welche auf die Oberfläche registriert wird, welche aus einem präoperativen Datensatz gewonnen wurde. Solche Techniken benötigen zusätzliche Hardware und sind mit einem gewissen Zeitaufwand verbunden, um die Messdaten durch Bedienung des Zeigerinstruments zu erfassen.

Um flächiger, schneller und ohne zusätzliche Hardware die Oberfläche zu erfassen, wurden Verfahren vorgeschlagen, welche stereoskopische Bilddaten eines robotischen Visualisierungssystems (z.B. Operationsmikroskop) nutzen, um daraus die Oberfläche des Patientenkopfes zu bestimmen. Siehe z.B. US7561733B2 oder DE102014210051A1. Diese Verfahren setzen jedoch voraus, dass das robotische Visualisierungssystems bereits auf den Patienten ausgerichtet ist, sodass die stereoskopischen Bilddaten die Zielregion abbilden, welche eine Registrierung ermöglicht. Dies ist jedoch im Normalfall nicht gegeben, da diese Information erst nach der Patientenregistrierung vorliegen würde. Deshalb kann ein händisches Ausrichten notwendig sein, was wiederum aufwendig und fehleranfällig ist.

Die US 5 795 294 A betrifft ein Verfahren zur Korrelation unterschiedlicher Koordinatensysteme in der computergestützten, stereotaktischen Chirurgie. Bei dem Verfahren werden ohne zusätzliche Referenzmarker topographische Teilinformationen mittels eines auf einem Trägersystem montierten Operationsmikroskops erfasst und mit einem geeigneten Korrelationsalgorithmus räumlich mit den vor der Operation generierten Diagnosedaten korreliert.

Die US 2007/270690 A1 betrifft eine medizinische Registriereinrichtung. Die Registriereinrichtung umfasst eine Lokalisierungseinrichtung, mit der die räumliche Position von Behandlungsgeräten, Behandlungshilfsmittel, Patienten oder Patiententeilen erfasst werden kann. Die Lokalisierungseinrichtung umfasst alle Mittel, die eine räumliche Position des Patienten, der Behandlungsgeräte oder der Behandlungshilfsmittel (z.B. eine Position im dreidimensionalen Raum eines Instrumentes oder seiner Spitze) ermitteln können. Die Registriereinrichtung kann ferner eine Datenverarbeitungseinheit umfassen, die erkannte Positionen von Patienten oder Patiententeilen den entsprechenden Punkten eines erfassten Patientenbildsatzes zuordnet. Die Registriereinrichtung kann eine Abstandsmesseinrichtung umfassen, deren räumliche Position von der Lokalisierungseinrichtung erfasst werden kann. Die Abstandsmesseinrichtung kann die Abstandsdaten für gemessene Punkte an die Datenverarbeitungseinheit übertragen.

### KURZE ZUSAMMENFASSUNG

Es besteht ein Bedarf für verbesserte Techniken, um die Bestimmung einer Transformationsvorschrift zwischen Bildern, die mittels eines robotischen Visualisierungssystems erfasst werden, und präoperativen Volumenbilddaten des Patienten zu ermöglichen. Insbesondere besteht ein Bedarf für Techniken, welche die oben genannten Nachteile und Einschränkungen vorbekannter Techniken beheben oder reduzieren.

Diese Aufgabe wird gelöst von den Merkmalen der unabhängigen Patentansprüche. Die Merkmale der abhängigen Patentansprüche definieren Ausführungsformen.

Mittels der hierin beschriebenen Techniken ist eine schnelle und effiziente Patientenregistrierung möglich. Das bedeutet, dass die Transformationsvorschrift zuverlässig und automatisiert bestimmt werden kann. Die Zielregion, für welche Messdaten mit Tiefenauflösung erfasst werden, um derart die Transformationsvorschrift zu bestimmen, kann automatisiert aufgefunden und erkannt werden.

Mittels der hierin beschriebenen Techniken ist es also möglich, präoperative Volumenbilddaten in einem Bezugskoordinatensystem zu registrieren, welches mit Bildern assoziiert ist, die mittels eines robotischen Visualisierungssystems erfasst werden.

Ein Computer-implementiert das Verfahren umfasst das Ansteuern eines robotischen Visualisierungssystems, um derart mindestens ein Suchbild aufzunehmen. Das mindestens eine Suchbild bildet einen Patienten zumindest teilweise ab. Außerdem umfasst das Verfahren auch das Bestimmen einer Anordnung einer Zielregion in dem mindestens einen Suchbild. Ferner umfasst das Verfahren das Ansteuern des robotischen Visualisierungssystems zum Aufnehmen von Messdaten mit Tiefenauflösung basierend auf der Anordnung der Zielregion in dem mindestens einen Suchbild. Die Messdaten sind dabei indikativ für eine Topographie einer Anatomie des Patienten. Die Messdaten ermöglichen das Bestimmen einer Transformationsvorschrift zwischen Bildern, die mittels des robotischen Visualisierungssystems aufgenommen werden, und präoperativen Volumenbilddaten des Patienten.

Die Zielregion kann einen Bereich des Patienten bezeichnen, für den die präoperativen Volumenbilddaten verfügbar sind. Die präoperativen Volumenbilddaten können also auch indikativ für die Topographie der Anatomie des Patienten im Bereich der Zielregion sein. Beispielsweise könnte die Zielregion einen Eingriffsbereich einer geplanten Operation bezeichnen. Die Zielregion könnte zum Beispiel einen Kopf eines Patienten umfassen. Die Zielregion können einen Teil des Kopfes eines Patienten umfassen.

Die Anordnung der Zielregion kann eine Position und/oder Orientierung und/oder Form und/oder Größe der Zielregion bezeichnen.

Durch das Aufnehmen des mindestens einen Suchbilds mittels des robotischen Visualisierungssystems kann also die Zielregion gesucht und erkannt werden.

Ein händisches Auffinden der Zielregion, beispielsweise durch manuelles Positionieren des robotischen Visualisierungssystems kann entfallen. Eine automatische Ausrichtung des robotischen Visualisierungssystems, so dass die Messdaten als Observablen für die Topographie der Anatomie des Patienten in der Zielregion erfasst werden können, wird basierend auf der bestimmten Anordnung der Zielregion in dem mindestens einen Suchbild ermöglicht.

Die präoperativen Volumenbilddaten könnten zum Beispiel CT-Bilddaten oder MR-Bilddaten sein. Es könnte sich auch um Positronen-Emissions-Tomographie-Bilddaten handelt.

Beispielsweise wäre es denkbar, dass ein einzelnes Suchbild mittels des robotischen Visualisierungssystems erfasst wird. Insbesondere könnte zum Beispiel ein Übersichtsbild mittels einer entsprechenden Kamera einer Übersichtsbild-Bildgebungseinheit erfasst werden, welche ein Objektiv verwendet, welches keine oder eine vergleichsweise geringe Vergrößerung bereitstellt. Das Suchbild könnte in einem solchen Fall die Zielregion und weitere umliegende Bereiche abbilden. Bei einer typischen Operation im Bereich des Kopfes des Patienten könnte zum Beispiel der Kopf des Patienten bis hinunter zum Brustbereich oder bis zum Torso oder Hüftbereich abgebildet werden.

In anderen Beispielen wäre es auch denkbar, dass mehrere Suchbilder aufgenommen werden, wobei jedes Suchbild einen unterschiedlichen Bereich abbildet. Es könnte ein Raster von Suchbildern erfasst werden.

Das robotische Visualisierungssystem wird angesteuert, um mindestens eine Suchbild-Sequenz aufzunehmen. Jede der mindestens einen Suchbild-Sequenz umfasst mehrere entsprechende Suchbilder.

Das bedeutet, dass die Suchbilder zum Beispiel einstufig oder zweistufig in ein oder zwei (oder mehr) Sequenzen erfasst werden können.

Beispielsweise könnte die jeweils mit der jeweiligen Suchbild-Sequenz assoziierte Suchregion von Suchbild-Sequenz zu Suchbild-Sequenz angepasst oder insbesondere verfeinert werden. Beispielsweise könnten die Suchbilder unterschiedlicher Suchbild-Sequenzen unterschiedliche Auflösungen aufweisen. Beispielsweise könnten die Suchbilder unterschiedlicher Suchbild-Sequenzen eine zunehmende Vergrößerung aufweisen.

Die Suchbilder einer Suchbild-Sequenz rastern eine entsprechende Suchregion ab. Dazu wird mindestens ein Motor des robotischen Visualisierungssystems während des Aufnehmens der mindestens einen Suchbild-Sequenz angesteuert, um das robotische Visualisierungssystem mehrfach umzupositionieren.

Beispielsweise könnte eine entsprechende Optik und Kamera einer Bildgebungseinheit, die zum Erfassen der Suchbilder verwendet wird, an einem Mikroskop-Kopf eines Operationsmikroskops angebracht sein, wobei der Mikroskop-Kopf über ein Stativ mit ein oder mehreren beweglichen Achsen im Raum positionierbar ist, zum Beispiel translatorisch und/oder rotatorisch.

Durch das Abrastern kann eine vergleichsweise große Suchregion abgedeckt werden. Gleichzeitig kann trotzdem eine relativ große Vergrößerung verwendet werden, so dass die Zielregion zuverlässig erkannt werden kann.

Einer ersten Suchbild-Sequenz der mindestens einen Suchbild-Sequenz ist eine erste Suchregion zugeordnet und einer zweiten Suchbild-Sequenz der mindestens einen Suchbild-Sequenz ist eine zweite Suchregion zugeordnet.

Die zweite Suchregion könnte beinhaltet sein in der ersten Suchregion. Die zweite Suchregion könnte auch teilweise verschieden von der ersten Suchregion sein.

Das robotische Visualisierungssystem wird dann angesteuert, um zuerst die erste Suchbild-Sequenz aufzunehmen und anschließend die zweite Suchbild-Sequenz aufzunehmen.

Das Verfahren umfasst das Auswerten der ein oder mehreren Suchbilder der ersten Suchbild-Sequenz. Basierend auf diesem Auswerten werden dann Werte für mindestens einen Bildgebungsparameter des robotischen Visualisierungssystems, der zum Aufnehmen der zweiten Suchbild-Sequenz verwendet wird, bestimmt.

Als allgemeine Regel können Bildgebungsparameter die räumliche Anordnung des zur Erfassung der entsprechenden Bilder verwendeten Bildgebungseinheit des robotischen Visualisierungssystems und/oder auch die Parametrisierung der Bildgebungskette (Zoom, Fokus, Licht, Belichtungsparameter, usw.) bezeichnen. Bildgebungsparameter können also solche Parameter sein, welche das Erscheinungsbild eines Bildes und/oder das Sichtfeld eines Bildes beeinflussen.

Mittels solcher Techniken ist es also möglich, eine situationsspezifische Anpassung von Werten für mindestens einen Bildgebungsparameter vorzunehmen. Je nach konkreter Situation im Zusammenhang mit der Visualisierung des jeweiligen Patienten können die Werte für den mindestens einen Bildgebungsparameter geeignet angepasst werden, so dass die Zielregion zuverlässig mittels der Suchbilder der zweiten Suchbilder Sequenz aufgefunden werden kann.

Beispielsweise könnte das Auswerten der ein oder mehreren Suchbilder der ersten Suchbild-Sequenz das Bestimmen einer Anordnung eines ortsfest zum patientenfixierten Markers in den ein oder mehreren Suchbildern der Suchbild-Sequenz umfassen. Dann könnten die Werte für den mindestens einen Bildgebungsparameter basierend auf der Anordnung des ortsfest zum Patienten fixierten Markers bestimmt werden.

Beispielsweise könnte die Anordnung der zweiten Suchregion in Bezug auf die erste Suchregion durch Einstellung der Sichtfelder der Suchbilder der zweiten Suchregion bestimmt werden. Beispielsweise könnte die relative Anordnung des Markers in Bezug auf die Zielregion grundsätzlich vorbekannt sein. Der Marker könnte zum Beispiel mittels einer Klemme oder anderen Fixierungsmitteln am Patienten angebracht sein, versetzt zur Zielregion. Dieser Versatz kann bekannt sein. Dann könnte die zweite Suchregion relativen Bezug auf die erste Suchregion bzw. auf den in den Suchbildern der ersten Suchregion aufgefundenen Marker angeordnet sein.

Eine weitere beispielhafte Implementierung der Auswertung der ein oder mehreren Suchbilder der ersten Suchbild-Sequenz kann zum Beispiel das Ermitteln von Werten für ein oder mehrere Beleuchtungsparameter für das Aufnehmen der zweiten Suchbild-Sequenz umfassen. So wäre es zum Beispiel denkbar, dass Helligkeitshistogramme für die ein oder mehreren Suchbilder der ersten Suchbild-Sequenz ausgewertet werden und dann die Belichtungszeitdauer und/oder die Helligkeit einer Beleuchtung für das Aufnehmen der ein oder mehreren Suchbilder der zweiten Suchbild-Sequenz eingestellt wird, so dass die Helligkeit-Histogrammen für die ein oder mehreren Suchbilder der zweiten Suchbild-Sequenz mit einer Zielvorgabe übereinstimmen. Derart kann sichergestellt werden, dass der Kontrast, mit dem bestimmte anatomische Merkmale oder beispielsweise Hautgewebe abgebildet wird, in den ein oder mehreren Suchbildern der zweiten Suchbild-Sequenz Details zum Auffinden der Zielregion abbildet.

In weiteren Beispielen wäre es auch denkbar, dass basierend auf dem Auswerten der ein oder mehreren Suchbilder der ersten Suchbild-Sequenz bestimmt wird, ob ein oder mehrere Suchbilder der ersten Suchbild-Sequenz für die zweite Suchbild-Sequenz wiederverwendet werden können.

Wird zum Beispiel die zweite Suchregion für die zweite Suchbild-Sequenz derart bestimmt, dass dieser eine Überlappung mit der ersten Suchregion der ersten Suchbild-Sequenz aufweist, so könnte in einem entsprechenden Überlappungsbereich eine Wiederverwendung von Suchbildern, die bereits für die erste Suchbild-Sequenz aufgenommen wurden, auch für die zweite Suchbild-Sequenz erfolgen. Derart kann die für das Erfassen der Suchbilder benötigte Zeitspanne reduziert werden.

Allgemein formuliert ist es denkbar, dass zumindest ein Suchbild sowohl Teil der ersten Suchbild-Sequenz ist, wie auch Teil der zweiten Suchbild-Sequenz ist.

Beispielsweise wäre es denkbar, dass die zweite Suchbild-Sequenz zum Erkennen von Hautgewebe des Patienten in den ein oder mehreren Suchbildern der zweiten Suchbild-Sequenz ausgewertet wird. Dann könnte die Anordnung der Zielregion basierend auf dem Erkennen des Hautgewebes bestimmt werden.

Typischerweise ist der Patient größtenteils abgedeckt, beispielsweise mit einem Operationstuch. Lediglich die Zielregion und unmittelbar umliegende Bereiche können freiliegenden sein. Deshalb kann mittels der Erkennung von Hautgewebe die Zielregion erkannt werden.

Das Verfahren kann das Empfangen von Trackingdaten von einem Trackingsystem umfassen. Das Trackingsystem kann zum robotischen Visualisierungssystem kollokiert sein. Das bedeutet, dass das robotische Visualisierungssystem und das Trackingsystem in einem gemeinsamen Referenzkoordinatensystem operieren können bzw. das Trackingsystem dazu verwendet werden kann, um die Anordnung des robotischen Visualisierungssystems (und damit auch die Pose bzw. das Sichtfeld von mittels des robotischen Visualisierungssystems erfassten Messdaten oder Bildern) im Referenzkoordinatensystem zu bestimmen. Das Trackingsystem kann auch eingerichtet sein, um die Anordnung (das heißt die Position und/oder Orientierung) anderer Marker im Referenzkoordinatensystem zu bestimmen. Das Referenzkoordinatensystem kann in Bezug auf den Operationssaal bestimmt sein.

Die Trackingdaten könnten beispielsweise Anordnung eines ortsfest zum Patienten fixierten Markers in Bezug auf das robotische Visualisierungssystem beschreiben. Der Marker kann z.B. durch passive maschinenlesbare Zeichen oder aktive maschinenlesbare Lichtquellen implementiert sein.

Entsprechend könnte das Verfahren weiterhin das Bestimmen von Werten für mindestens einen Bildgebungsparameter des robotischen Visualisierungssystems, der zum Aufnehmen der mindestens einen Suchbild-Sequenz verwendet wird, basierend auf den Trackingdaten umfassen.

Beispielsweise könnte einer Anordnung einer entsprechenden Suchregion, die von der mindestens einen Suchbild-Sequenz abgedeckt wird, basierend auf den Trackingdaten bestimmt werden. Wird zum Beispiel mittels des Trackingsystems der Marker erkannt, so kann die relative Positionierung des Markers in Bezug auf die Zielregion vorbekannt sein.

Allgemein kann es also möglich sein, dass Werte für mindestens einen Bildgebungsparameter des robotischen Visualisierungssystems, der zum Aufnehmen der mindestens einen Suchbild-Sequenz verwendet wird, basierend auf Vorwissen bestimmt wird. Beispielsweise könnte eine Helligkeit einer Beleuchtung als beispielhafter Bildgebungsparameter eingestellt werden. Es könnte eine Belichtungszeit als beispielhafter Bildgebungsparameter eingestellt werden. Es könnte eine Anordnung von entsprechenden Suchregionen bestimmt werden. Es könnte eine Wiederverwendung von Suchbildern zwischen unterschiedlichen Suchbild-Sequenzen bestimmt werden.

Solches Vorwissen kann zum Beispiel die (grobe) Anordnung des Patienten in Bezug auf das robotische Visualisierungssystem betreffen. Alternativ oder zusätzlich könnte solches Vorwissen auch die Anordnung der Zielregion in Bezug zu einem ortsfest zum Patienten fixierten Marker betreffen. Beispielsweise kann der Patient nämlich in Bezug auf das robotische Visualisierungssystem grob vor-positioniert sein. Beispielsweise kann der Patient auf einer Patientenliege angeordnet werden, wobei die Anordnung der Patientenliege in Bezug auf das robotische Visualisierungssystem bekannt ist. Derart könnte in einem ersten Schritt eine grobe Ausrichtung des robotischen Visualisierungssystem auf die (vermutete) Position des Patienten erfolgen und anschließend basierend auf diesem Vorwissen die Suchbilder der mindestens einen

Suchbild-Sequenz erfasst werden. Entsprechendes kann auch für einen ortsfest zum Patienten fixierten Marker gelten.

Das Verfahren könnte ferner das Auswerten von bereits erfassten Suchbildern der mindestens einen Suchbild-Sequenz während des Aufnehmens der mindestens einen Suchbild-Sequenz umfassen. In Abhängigkeit von dem Auswerten könnte dann selektiv das Aufnehmen der mindestens einen Suchbild-Sequenz abgebrochen werden, das heißt es kann darauf verzichtet werden, weitere Suchbilder zu erfassen. Alternativ oder zusätzlich wäre es auch denkbar, in Abhängigkeit von dem Auswerten Werte für mindestens einen Bildgebungsparameter des robotischen Visualisierungssystems, welcher zum Aufnehmen der mindestens einen Suchbild-Sequenz verwendet wird, anzupassen.

Werden also mehrere Suchbilder erfasst, so kann fortlaufend überprüft werden, ob es notwendig ist, dass weitere Suchbilder erfasst werden (oder ob beispielsweise die Zielregion bereits erkannt wurde und damit das weitere Erfassen von Suchbildern entbehrlich ist). Ferner könnte überprüft werden, ob die Werte für den mindestens einen Bildgebungsparameter geeignet eingestellt sind, oder ob eine Anpassung erfolgen soll, beispielsweise eine Anpassung der Helligkeit oder der Belichtungszeit usw.

Allgemein kann die Anordnung der Zielregion basierend auf einer Strukturerkennung von ein oder mehreren vorgegebenen Strukturen in dem mindestens einen Suchbild bestimmt werden. So könnte beispielsweise die Struktur von Hautgewebe in dem mindestens einen Suchbild erkannt werden. Alternativ oder zusätzlich könnte die Struktur bestimmter Anatomiemerkmale (beispielsweise Nase, Ohr, Mund, Auge, usw.) in dem mindestens einen Suchbild erkannt werden. Geeignete Algorithmen können eingesetzt werden. Die konkrete Implementierung eines solchen Algorithmus zur Bestimmung der Anordnung der Zielregion ist aber nicht wesentlich für die Implementierung der hierin beschriebenen Techniken und es kann auf vorbekannte Implementierungen zurückgegriffen werden.

Zum Aufnehmen des mindestens einen Suchbilds kann beispielsweise eine Übersichtsbild-Bildgebungseinheit des robotischen Visualisierungssystems und/oder eine Mikroskopie-Bildgebungseinheit des robotischen Visualisierungssystems angesteuert werden. Das bedeutet in anderen Worten, dass das mindestens eine Suchbild zum Beispiel ein Übersichtsbild mit vergleichsweise geringer Vergrößerung aufweisen kann und/oder auch ein Mikroskopiebild mit vergleichsweise großer Vergrößerung aufweisen kann.

Die Messdaten können beispielsweise mit einer Messmodalität erfasst werden, welche aus der folgenden Gruppe ausgewählt ist: stereoskopische Bildgebung; Lichtlaufzeit-Messung; strukturierte Beleuchtung; und Defokus-Tiefen Schätzung.

Bei der stereoskopischen Bildgebung werden mittels zweier Kameras, die denselben Bereich mit unterschiedlichen Posen abbilden, Informationen über die Topographie gewonnen.

Bei der Lichtlaufzeit-Messung (engl. time-of-flight) kann mittels kurzer Lichtpulse und der entsprechenden Überwachung der Laufzeit Information über die Topographie gewonnen werden.

Bei der strukturierten Beleuchtung wird ein vorgegebenes Muster, beispielsweise ein Linienmuster, auf eine zu vermessende Oberfläche projiziert. Die Verzerrung des vorgegebenen Musters kann dann dazu verwendet werden, um Information über die Topographie zu gewinnen.

Bei der Defokus-Tiefenschätzung kann über die lokale Verwendung eines Autofokussierung Verfahrens Information über die Topographie gewonnen werden.

Als allgemeine Regel wäre es denkbar, dass die Messdaten zumindest ein Suchbild umfassen. Das bedeutet, dass es nicht unbedingt notwendig ist, dass die Messdaten komplett getrennt von den Suchbildern erfasst werden.

Das Verfahren könnte ferner das Filtern der Messdaten umfassen. Beispielsweise könnten dabei Datenelemente aus den Messdaten entfernt werden, welche nicht Hautgewebe des Patienten abbilden. Beispielsweise könnten Bereiche aus den Messdaten entfernt werden, die Operationstücher oder Marker oder Operationsbesteck abbilden. Dadurch wird eine fehlerhafte Registrierung der Messdaten auf die präoperativen Volumenbilddaten vermieden.

Vor dem Aufnehmen des mindestens einen Suchbilds kann der Motor des robotischen Visualisierungssystems angesteuert werden, um das robotische Visualisierungssystem in einer vorgegebenen Referenzanordnung anzuordnen. Beispielsweise könnte die vorgegebene Referenzanordnung in Bezug auf einen Operationstisch definiert sein. Die vorgegebene Referenzanordnung könnte zum Beispiel derart definiert sein, dass ein Bereich des Operationstisch, auf dem der Kopf des Patienten anzuordnen ist, im Sichtfeld der Bildgebungseinheit des robotischen Visualisierungssystems liegt, welche zum Aufnehmen ersten des mindestens einen Suchbilds verwendet wird.

Es wäre denkbar, dass das Verfahren ferner das Setzen von mindestens einen Bildgebungsparameter des robotischen Visualisierungssystems vor dem Aufnehmen des mindestens einen Suchbilds auf einen vorgegebenen Wert umfasst. Dies könnte beispielsweise eine Einstellung einer Zielvergrößerung für einen Zoom-Faktor einer entsprechenden Bildgebungseinheit des robotischen Visualisierungssystems umfassen. Eine bestimmte Helligkeit einer Beleuchtung könnte ausgewählt werden. Es wäre denkbar, dass die Belichtungszeit auf einen bestimmten Wert eingestellt wird.

Ein hierin beschriebenes Verfahren kann basierend auf einem vordefinierten Steuerungsskript automatisiert durchgeführt werden. Beispielsweise kann mittels eines einzigen Benutzerbefehls (z.B. "one-click") das Steuerungsskript ausgelöst werden. Derart kann eine besonders einfache und schnelle Erfassung von Messdaten, die anschließend das Bestimmen der Transformationsvorschrift ermöglicht, erreicht werden; ein händisches Umpositionieren des robotischen Visualisierungssystems kann entbehrlich sein.

Beispielsweise könnte ein solches Steuerungsskript ein Analyse-Modul zur Auswertung von Bilddaten oder Messdaten umfassen. Außerdem könnte das Steuerungsskript zumindest ein Positionierungs-Modul zur Positionierung des robotischen Visualisierungssystems umfassen. Eine solche Positionierung könnte zum Beispiel dazu verwendet werden, um mittels einer Rasterung Suchbilder einer Suchbild-Sequenz zu erfassen, die eine Suchregion abdecken, die größer ist als das Sichtfeld eines einzelnen Suchbilds. Das Analyse-Modul könnte zum Beispiel Bilddaten auswerten, um Hautgewebe aufzufinden. Das Analyse-Modul könnte zum Beispiel Bilddaten auswerten, um einen Marker, der ortsfest in Bezug auf den patientenfixierten ist, zu erkennen.

Das Analyse-Modul kann zum Beispiel ein oder mehrere maschinengelernte Algorithmen umfassen. Beispielsweise könnten künstliche neuronale Netzwerke verwendet werden, die vorgegebene Strukturen oder Objekte in Bildern erkennen. Dazu könnte zum Beispiel ein Faltungsnetzwerk (engl. convolutional neural network) verwendet werden. Entsprechendes Training kann zum Beispiel mittels manueller Annotation von entsprechenden Trainings-Bilddaten oder Trainings-Messdaten ermöglicht werden.

Andererseits wäre es denkbar, dass das Positionierungs-Modul keine maschinengelernten Algorithmen umfasst. Es kann ein händisch parametrisierter Algorithmus eingesetzt werden. Dies kann insbesondere deshalb erstrebenswert sein, weil derart für jeden Eingangsparameter die neue Position des Positionierungs-Modulen einer Validierungsphase validiert werden kann, um unkontrollierte oder gefährliche Bewegungen des robotischen Visualisierungssystems zu vermeiden.

Das Verfahren kann weiterhin das Erzeugen eines Topographie-Datensatzes basierend auf den Messdaten umfassen. Das bedeutet, dass die Messdaten ausgewertet werden können, zum Beispiel mittels eines Analyse-Moduls wie voranstehend beschrieben.

Das Verfahren kann ferner das Durchführen einer Registrierung des Topographie-Datensatzes mit den präoperativen Volumenbilddaten umfassen. Das bedeutet, dass korrespondierende Punkte in dem Topographie-Datensatz und den präoperativen Volumenbilddaten aufgefunden werden können. Basierend auf der relativen Anordnung solcher korrespondierenden Punkte zueinander kann dann die Transformationsvorschrift bestimmt werden. Allgemein kann basierend auf der Registrierung die Transformationsvorschrift bestimmt werden.

Eine Datenverarbeitungseinheit ist eingerichtet, um ein robotisches Visualisierungssystem anzusteuern, um derart mindestens ein Suchbild aufzunehmen. Das mindestens eine Suchbild bildet einen Patienten zumindest teilweise ab. Außerdem ist die Datenverarbeitungseinheit eingerichtet, um die Anordnung einer Zielregion in dem mindestens einen Suchbild zu bestimmen und basierend auf dieser Anordnung der Zielregion in dem mindestens einen Suchbild das robotische Visualisierungssystem anzusteuern, um Messdaten mit Tiefenauflösung aufzunehmen. Diese Messdaten sind indikativ für eine Topographie einer Anatomie des Patienten. Derart wird das Bestimmen einer Transformationsvorschrift zwischen mittels des robotischen Visualisierungssystems aufgenommenen Bildern und präoperativen Volumenbilddaten des Patienten ermöglicht. Insbesondere ist die Datenverarbeitungseinheit eingerichtet, das oben beschrieben Verfahren auszuführen.

Ein System umfasst die Datenverarbeitungseinheit und das robotische Visualisierungssystem.

Ein Computerprogramm oder ein Computerprogramm-Produkt oder ein computerlesbares Speichermedium umfasst Programmcode. Der Programmcode kann von einem Prozessor geladen und ausgeführt werden. Dies bewirkt, dass der Prozessor ein Verfahren ausführt. Das Verfahren umfasst das Ansteuern eines robotischen Visualisierungssystems, um derart mindestens ein Suchbild aufzunehmen. Das mindestens eine Suchbild bildet einen Patienten zumindest teilweise ab. Außerdem umfasst das Verfahren auch das Bestimmen einer Anordnung einer Zielregion in dem mindestens einen Suchbild. Ferner umfasst das Verfahren das Ansteuern des robotischen Visualisierungssystems zum Aufnehmen von Messdaten mit Tiefenauflösung basierend auf der Anordnung der Zielregion in dem mindestens einen Suchbild. Die Messdaten sind dabei indikativ für eine Topographie einer Anatomie des Patienten. Die Messdaten ermöglichen das Bestimmen einer Transformationsvorschrift zwischen Bildern, die mittels des robotischen Visualisierungssystems aufgenommen werden, und präoperativen Volumenbilddaten des Patienten.

Die oben dargelegten Merkmale und Merkmale, die nachfolgend beschrieben werden, können nicht nur in den entsprechenden explizit dargelegten Kombinationen verwendet werden, sondern auch in weiteren Kombinationen oder isoliert, ohne den Schutzumfang der vorliegenden Erfindung zu verlassen.

### KURZE BESCHREIBUNG DER FIGUREN

FIG. 1 illustriert schematisch ein optisches Visualisierungssystem gemäß verschiedenen Beispielen.
FIG. 2 illustriert schematisch eine Datenverarbeitungseinheit gemäß verschiedenen Beispielen.
FIG. 3 ist ein Flussdiagramm eines beispielhaften Verfahrens.
FIG. 4 illustriert ein einzelnes Suchbild und eine Zielregion gemäß verschiedenen Beispielen.
FIG. 5 illustriert eine Suchbild-Sequenz und eine Zielregion gemäß verschiedenen Beispielen.

### DETAILLIERTE BESCHREIBUNG VON AUSFÜHRUNGSFORMEN

Die oben beschriebenen Eigenschaften, Merkmale und Vorteile dieser Erfindung sowie die Art und Weise, wie diese erreicht werden, werden klarer und deutlicher verständlich im Zusammenhang mit der folgenden Beschreibung der Ausführungsbeispiele, die im Zusammenhang mit den Zeichnungen näher erläutert werden.

Nachfolgend wird die vorliegende Erfindung anhand bevorzugter Ausführungsformen unter Bezugnahme auf die Zeichnungen näher erläutert. In den Figuren bezeichnen gleiche Bezugszeichen gleiche oder ähnliche Elemente. Die Figuren sind schematische Repräsentationen verschiedener Ausführungsformen der Erfindung. In den Figuren dargestellte Elemente sind nicht notwendigerweise maßstabsgetreu dargestellt. Vielmehr sind die verschiedenen in den Figuren dargestellten Elemente derart wiedergegeben, dass ihre Funktion und genereller Zweck dem Fachmann verständlich wird. In den Figuren dargestellte Verbindungen und Kopplungen zwischen funktionellen Einheiten und Elementen können auch als indirekte Verbindung oder Kopplung implementiert werden. Eine Verbindung oder Kopplung kann drahtgebunden oder drahtlos implementiert sein. Funktionale Einheiten können als Hardware, Software oder eine Kombination aus Hardware und Software implementiert werden.

Nachfolgend werden Techniken beschrieben, welche es ermöglichen, einer Transformationsvorschrift zu bestimmen, die Bilddaten, die mittels eines robotischen Visualisierungssystems erfasst werden (nachfolgend einfach OP-Mikroskop genannt) abzubilden bzw. zu überführen auf präoperativen Volumenbilddaten; auch die umgekehrte Abbildungsvorschrift kann von der Transformationsvorschrift erfasst werden.

Dazu wird eine Zielregion automatisch erkannt bzw. deren Anordnung automatisch bestimmt, basierend auf ein oder mehreren Suchbildern. Es ist dann möglich, für diese Zielregion Messdaten mit Tiefenauflösung zu bestimmen, welche auf die präoperativen Volumenbilddaten registriert werden können, um aus dieser Registrierung die Transformationsvorschrift abzuleiten.

Auf Grundlage dieser Transformationsvorschrift können dann unterschiedliche Anwendungen ermöglicht werden. Beispielsweise könnte eine Assistenzfunktionalität an einen Operateur basierend auf den präoperativen Volumenbilddaten und der Transformationsvorschrift bereitgestellt werden. Beispielsweise könnten die präoperativen Volumenbilddaten oder Teile davon in ein Sichtfeld des OP-Mikroskops oder in mittels des OP-Mikroskops erfasste Bilddaten ortsgetreu eingeblendet werden. Es könnten bestimmte Bereiche, die in den präoperativen Volumenbilddaten gekennzeichnet sind, in mittels des OP-Mikroskops erfassten Bilddaten hervorgehoben werden. Eine Navigations-Assistenzfunktionalität, die den operativen Eingriff führt, kann ermöglicht werden.

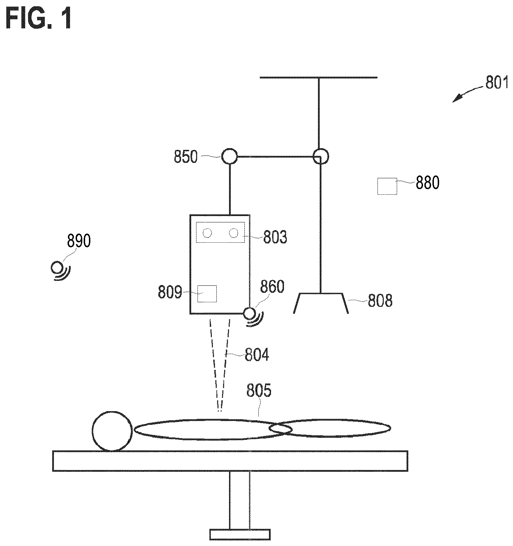

FIG. 1 zeigt schematisch ein OP-Mikroskop 801 für die Chirurgie. Das Operationsmikroskop 801 verfügt im dargestellten Beispiel über ein Okular 803. Der Operateur kann durch das Okular 803 vergrößerte Bilder von einem Objekt, das sich in einem Sichtfeld 804 des Operationsmikroskops 801 befindet, betrachten. Im dargestellten Beispiel handelt es sich um einen Patienten 805, der auf einer Patientenliege liegt.

Alternativ oder zusätzlich zu einem optischen Okular könnte auch eine Kamera 809 vorgesehen sein, die Bilder an einen Bildschirm überträgt (digitales Operationsmikroskop).

Allgemein formuliert kann das OP-Mikroskop 801 ein oder mehrere der Bildgebungseinheit umfassen, die eingerichtet sind, um digitale Bilder aufzunehmen. Beispiele für Bildgebungseinheit wären zum Beispiel eine Mikroskopie-Bildgebungseinheit und eine Übersichtsbild-Bildgebungseinheit. Die Mikroskopie-Bildgebungseinheit könnte ein oder zwei oder mehr Kameras beinhalten; beispielsweise könnte eine stereoskopische Bildgebung mit mehreren Kameras möglich sein.

Es ist auch eine Bedieneinrichtung 808 als Mensch-Maschine-Schnittstelle vorgesehen, die beispielsweise als Handgriff oder Fußschalter ausgebildet sein kann. In der dargestellten Ausführungsform der FIG. 1 ist es ein Handgriff. Durch die Bedieneinrichtung 808 kann das an Traversen 850 befestigte Okular 803 bewegt werden. Es können Motoren vorgesehen sein, um die Bewegung basierend auf Steuerdaten automatisch durchzuführen, gemäß einer entsprechenden Einstellung des OP-Mikroskops. Die Motoren könnten die durch den Handgriff 808 veranlasste Bewegung auch unterstützen.

Weiter ist für das OP-Mikroskop 801 eine Steuergerät 880 vorgesehen, das den Betrieb des OP-Mikroskops 801 und die Anzeige von Bildern sowie zusätzlichen Informationen und Daten im Okular 803 steuert. Das Steuergerät 880 kann eine Interaktion mit dem Operateur durchführen.

Das OP-Mikroskop 801 kann auch über ein oder mehrere weitere Sensoren 860 verfügen, etwa eine Umfeldkamera, einen Lichtlaufzeitsensor (TOF-Kamera), eine Bildaufnahmeeinrichtung zur Aufnahme von Bildern, die bei einer strukturierten Beleuchtung erfasst werden, usw. mittels solcher Sensoren 860 können Messdaten erfasst werden, die beispielsweise eine Tiefenauflösung aufweisen. Das bedeutet, dass die Tiefenauflösung den Abstand zwischen dem entsprechenden Sensor 860 und dem Patienten 805 beschreiben kann.

Eine weitere Implementierung eines solchen Sensors 860 beträfe ein internes Trackingsystem. Mittels eines solchen internen Trackingsystems können zum Beispiel Marker, die relativen Bezug auf den Patienten fixiert sind und maschinenlesbare ausgebildet sind, erkannt werden. Marker werden manchmal auch als Targets bezeichnet. Insbesondere kann die relative Anordnung der Marker in Bezug auf das OP-Mikroskop 801 bestimmt werden.

FIG. 1 illustriert auch ein externes Trackingsystem 890. Dieses ist mit dem OP-Mikroskop 801 kollokiert. Es ist möglich, dass Trackingdaten 890 vom Trackingsystem 890 an das Steuergerät 880 übertragen werden, wobei die Trackingdaten zum Beispiel indikativ für die Anordnung der Kamera 809 oder des Okulars 803 oder allgemein des Sichtfelds 804 einer Bildgebungseinheit im Raum sind, beispielsweise in Bezug auf ortsfest in Bezug auf den Patienten 805 positionierte Marker oder ein im Raum feststehendes Referenzkoordinatensystem.

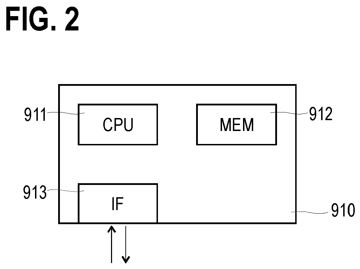

FIG. 2 illustriert Aspekte im Zusammenhang mit einer Datenverarbeitungseinheit 910. Die Datenverarbeitungseinheit 910 könnte zum Beispiel von einem Computer implementiert werden. Die Datenverarbeitungseinheit 910 könnte zum Beispiel das Steuergerät 880 des OP-Mikroskops 801 implementieren (vergleiche FIG. 1) oder könnte auch separat vom OP-Mikroskop 801 ausgebildet sein (und dann zum Beispiel mit dem Steuergerät 880 kommunizieren).

Die Datenverarbeitungseinheit 910 umfasst eine Recheneinheit 911 und einen Speicher 912 und eine Kommunikationsschnittstelle 913. Die Recheneinheit 911 kann zum Beispiel Programmcode aus dem Speicher 912 laden und ausführen. Die Recheneinheit 911 kann über die Kommunikationsschnittstelle 913 mit anderen Geräten, Knoten oder Vorrichtungen kommunizieren. Beispielsweise könnten Trackingdaten von einem Trackingsystem empfangen werden. Es wäre möglich, präoperativen Volumenbilddaten zu empfangen, zum Beispiel von einem Bild-Archiv-System oder einer anderen Datenbank. Es könnten Benutzereingaben von einer Mensch-Maschine-Schnittstelle empfangen werden.

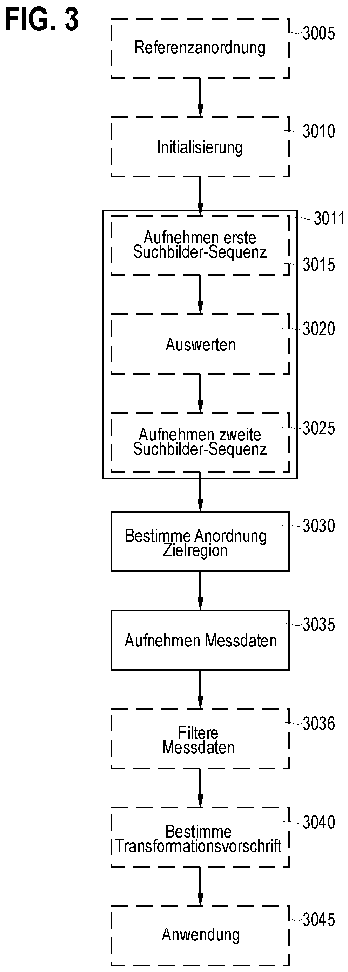

Wenn die Recheneinheit 911 Programmcode ausführt, der zum Beispiel von dem Speicher 912 geladen wird, so kann dies bewirken, dass die Recheneinheit 911 Techniken ausführt, wie sie hierin beschrieben sind, etwa: Aufnehmen von Suchbildern oder Messdaten; Auswerten von Suchbildern oder Messdaten; Bestimmen einer Anordnung einer Zielregion in Suchbildern; Ansteuern des OP-Mikroskops 801 um Bildder, Suchbilder, oder Messdaten zu erfassen und/oder eine bestimmte Position des Sichtfelds 804 zu bewirken; Bereitstellen von Steuerdaten, um eine Assistenzfunktionalität für einen Operateur zu ermöglichen; Bestimmen einer Transformationsvorschrift zwischen mittels des OP-Mikroskops 801 aufgenommenen Bilddaten und präoperativen Volumenbilddaten, beispielsweise basierend auf einer entsprechenden Registrierung zwischen Messdaten mit Tiefenauflösung sowie den präoperativen Volumenbilddaten. Verschiedene Funktionalitäten, die von der Recheneinheit 911 basierend auf sprechenden Programmcode bereitgestellt und implementiert werden können, werden nachfolgend im Zusammenhang mit dem Verfahren in FIG. 3 beschrieben.

FIG. 3 ist ein Flussdiagramm eines beispielhaften Verfahrens. Beispielsweise könnte das Verfahren der FIG. 3 von einer Datenverarbeitungseinheit wie zum Beispiel der Datenverarbeitungseinheit 910 aus FIG. 2 ausgeführt werden. Insbesondere könnte das Verfahren aus FIG. 3 von einer Recheneinheit, beispielsweise einem Prozessor, ausgeführt werden, wenn dieser Programmcode lädt und ausführt.

Das Verfahren der FIG. 3 dient dazu, die Bestimmung einer Transformationsvorschrift zwischen Bildern, die mittels eines OP-Mikroskops aufgenommen werden, und präoperativen Volumenbilddaten - zum Beispiel CT-Bilddaten oder MRT-Bilddaten - zu ermöglichen. In anderen Worten dient das Verfahren der FIG. 3 also zur Kalibration der Positionierung des Patienten in Bezug auf das OP-Mikroskop sowie relativ zu den präoperativen Volumenbilddaten. Insbesondere unterstützt das Verfahren der FIG. 3 eine automatisierte Erfassung von Messdaten mit Tiefenauflösung durch das OP-Mikroskop, so dass diese Messdaten dann auf die präoperativen Volumenbilddaten registriert werden können. Basierend auf dieser Registrierung kann dann die Transformationsvorschrift bestimmt werden, die Transformationsvorschrift könnte aber auch in einem nachgelagerten Verfahren, beispielsweise von einer anderen Datenverarbeitungsanlage bestimmt werden, etwa von einem Steuergerät eines Trackingsystems.

Beispielsweise wäre es denkbar, dass das Verfahren gemäß FIG. 3 basierend auf einem automatisierten Steuerungsskript ausgeführt wird. Das bedeutet, dass die verschiedenen Verfahrensschritte automatisiert nacheinander von einem Computerprogramm abgearbeitet werden können. Dies weist den Vorteil auf, dass der Benutzer beispielsweise das entsprechende Steuerungsskript lediglich durch eine einzelne Mensch-Maschinen-Interaktion auslösen kann, und keine manuellen Interaktionen während der Abarbeitung der entsprechenden Verfahrensschritte mehr notwendig ist.

Optionale Boxen sind in FIG. 3 mit gestrichelten Linien dargestellt.

Zunächst kann in Box 3005 einen Motor des OP-Mikroskops angesteuert werden, um das OP-Mikroskop in einer vorgegebenen Referenzanordnung anzuordnen. Beispielsweise könnte diese Referenzanordnung bewirken, dass das Sichtfeld des OP-Mikroskops auf einen Kopfbereich eines Patienten ausgerichtet ist, wenn dieser auf einer entsprechenden Liege angeordnet ist.

Anschließend ist es in Box 3010 optional möglich, eine Initialisierung von ein oder mehreren Bildgebungsparametern durchzuführen. Das bedeutet, dass es möglich wäre, mindestens einen Bildgebungsparameter des OP-Mikroskops vor dem Aufnehmen von Suchbildern auf einen vorgegebenen Wert zu setzen. Beispielsweise könnte die Helligkeit einer Beleuchtung initialisiert werden oder es wäre denkbar, dass ein Vergrößerungsfaktor eines Zooms eingestellt wird. Es könnte ein vorgegebener Fokus gewählt werden. Ein bestimmtes Sichtfeld könnte eingestellt werden.

Manchmal können Box 3010 und Box 3005 auch zusammen implementiert werden. Beispielsweise wenn das Bildgebungsparameter die Positionierung einer entsprechenden Bildgebungseinheit des OP-Mikroskops in Box 3010 eingestellt wird.

Anschließend erfolgt in Box 3011 das Aufnehmen von mindestens einem Suchbild, welches den Patienten zumindest teilweise abbildet. Dazu kann das OP-Mikroskop oder genau genommen eine entsprechende Bildgebungseinheit oder ein entsprechendes Steuergerät angesteuert werden. Ein Befehl zum Erfassen des entsprechenden mindestens einen Suchbilds kann gesendet werden.

Zum Beispiel könnte eine Übersichtsbild-Bildgebungseinheit des OP-Mikroskops und/oder eine Mikroskopie-Bildgebungseinheit des OP-Mikroskops zum Aufnehmen des mindestens einen Suchbilds angesteuert werden. Das bedeutet also, dass zum Beispiel ein oder mehrere Übersichtsbilder mit geringer Vergrößerung und/oder ein oder mehrere Mikroskopiebilder mit großer Vergrößerung verwendet werden können, um das mindestens einen Suchbild zu implementieren.

Es gibt unterschiedliche Implementierungsvarianten für Box 3011. Bevor diese Implementierungsvarianten nachfolgend im Detail diskutiert werden, wird zunächst das nachfolgende Verfahren näher erläutert.

In Box 3030 wird eine Anordnung einer Zielregion in dem mindestens einen Suchbild bestimmt. Das bedeutet, dass eine Position und/oder Orientierung und/oder eine Ausdehnung einer Zielregion in dem mindestens einen Suchbild bestimmt wird. Ein Analyse-Modul kann eingesetzt werden. Dazu könnte zum Beispiel ein maschinengelernter Algorithmus eingesetzt werden. Ein solcher maschinengelernter Algorithmus könnte basierend auf Trainings-Suchbildern und händischen Annotationen von Zielregionen trainiert werden. Der maschinengelernte Algorithmus könnte zum Beispiel durch ein Faltungsnetzwerk (engl. convolutional neural network) implementiert werden.

Die Zielregion kann zum Beispiel einen Eingriffsbereich einer geplanten Operation bezeichnen. Die Zielregion kann einen Bereich des Körpers des Patienten bezeichnen, für den die präoperativen Volumenbilddaten erfasst wurden bzw. der durch die präoperativen Volumenbilddaten abgebildet wird.

Zum Beispiel könnte die Anordnung der Zielregion basierend auf einer Strukturerkennung von ein oder mehreren vorgegebenen Strukturen in dem mindestens einen Suchbild bestimmt werden. Es könnten zum Beispiel Hautgewebe oder Anatomiemerkmale erkannt werden. Es könnte aber auch ein maschinenlesbarer Marker erkannt werden.

Dann ist es möglich, in Box 3035 basierend auf dieser Anordnung der Zielregion Messdaten mit Tiefenauflösung zu erfassen. Dazu kann das OP-Mikroskop angesteuert werden, beispielsweise ein entsprechender Sensor, eine entsprechende Bildgebungseinheit oder ein zugeordnetes Steuergerät. Die Messdaten sind also indikativ für eine Topographie der Anatomie des Patienten im Bereich der Zielregion.

Beispielsweise könnten die Messdaten mit einer Messmodalität erfasst werden, die aus der Gruppe ausgewählt ist, die umfasst: stereoskopische Bildgebung; Lichtlaufzeit-Messung; strukturierte Beleuchtung; und Defokus-Tiefenschätzung.

Es wäre in manchen Beispielen auch möglich, dass die Messdaten zumindest eines des mindestens einen Suchbilds umfassen. Das bedeutet also, dass es denkbar wäre, dass bestimmte Bilder nicht nur als Suchbilder in Box 3011 verwendet werden, sondern auch im Rahmen der Messdaten in Box 3035 berücksichtigt werden, also eine doppelte Funktion erfüllen.

In Box 3036 wäre es optional möglich, die Messdaten zu filtern. Beispielsweise könnte eine Rauschunterdrückung durchgeführt werden. Hintergrund könnte entfernt werden. Es wäre möglich, Datenelemente zu entfernen, die nicht Hautgewebe des Patienten abbilden.

Derart kann es möglich sein, die Transformationsvorschrift zwischen mittels des OP-Mikroskops aufgenommenen Bildern und präoperativen Volumenbilddaten des Patienten zu bestimmen. Diese Transformationsvorschrift kann in optionaler Box 3040 bestimmt werden. Es wäre auch möglich, die Messdaten für eine spätere Bestimmung abzuspeichern oder an eine andere Datenverarbeitungseinheit zu übermitteln, so dass diese die Transformationsvorschrift bestimmt.

Wird die Transformationsvorschrift bestimmt, so kann dies wie folgt geschehen: basierend auf den Messdaten kann zunächst ein Topographie-Datensatz erzeugt werden. Das bedeutet, dass zum Beispiel ein Höhenprofil des Patientenkopfes oder Allgemein der Hautoberfläche des Patienten in der Zielregion bestimmt werden kann. Dann kann eine Registrierung dieses Topographie-Datensatzes mit den präoperativen Volumenbilddaten erfolgen. Die präoperativen Volumenbilddaten können nämlich insbesondere auch die Topographie der Hautoberfläche des Patientenkopfes abbilden. Basierend auf dieser Registrierung kann dann die Transformationsvorschrift bestimmt werden, beispielsweise unter Berücksichtigung von translatorischen und/oder rotatorischen Freiheitsgraden. Es können auch Verzerrungen berücksichtigt werden.

Optional kann in Box 3045 eine Anwendung ermöglicht werden, die auf dieser Transformationsvorschrift basiert. Beispielsweise könnte eine Computer-assistierte Operation ermöglicht werden, etwa im Rahmen von augmentierten Bildern, die mittels des OP-Mikroskops erfasst werden und Informationen aufweisen, die an Hand der präoperativen Volumenbilddaten bestimmt werden. Eine Navigations-Assistenzfunktionalität wird ermöglicht.

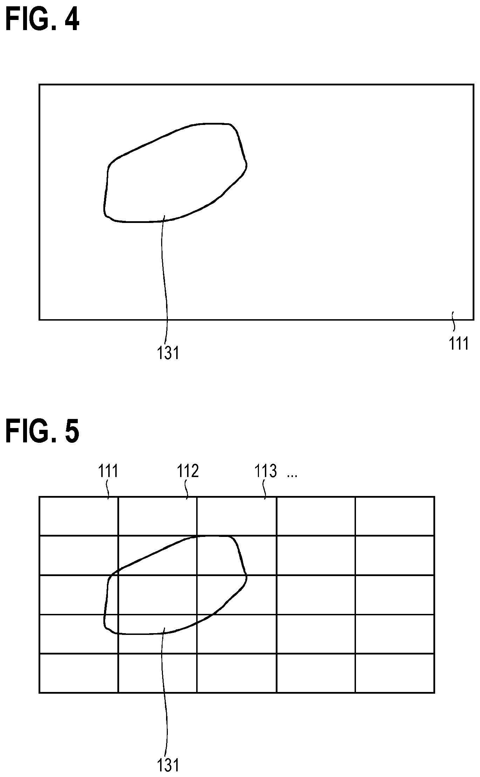

Nachfolgend werden Details im Zusammenhang mit Box 3011 beschrieben, nämlich mit dem Aufnehmen von mindestens einem Suchbild. In einer einfachen Variante wäre es möglich, ein einzelnes Suchbild aufzunehmen und in dem einzelnen Suchbild die Anordnung der Zielregion zu bestimmen. Ein solches Szenario ist in FIG. 4 dargestellt. In FIG. 4 ist ein Suchbild 111 dargestellt. Das Suchbild 111 bildet die Zielregion 131 ab. Beispielsweise könnte das Suchbild 111 mit einer Übersichtsbild-Bildgebungseinheit des OP-Mikroskops 801 (vergleiche FIG. 1) erfasst werden).

In einer anderen Variante könnten auch eine oder mehrere Suchbild-Sequenzen erfasst werden. Beispielsweise ist in FIG. 5 ein Szenario gezeigt, bei dem insgesamt 25 Suchbilder 111-113 (aus Gründen der Übersichtlichkeit sind nur die ersten drei Suchbilder mit Bezugszeichen versehen) erfasst werden. Beispielsweise könnte jedes dieser Suchbilder der Suchbild-Sequenz eine besonders hohe Auflösung aufweisen. Derart können auch große Vergrößerungen ermöglicht werden. Dies entspricht einem Abrastern einer entsprechenden Suchregion. Das Abrastern kann durch das Ansteuern eines Motors des OP-Mikroskops erreicht werden, so dass das OP-Mikroskop mehrfach umpositioniert wird, beispielsweise zwischen dem Erfassen der verschiedenen Suchbilder der Suchbild-Sequenz.

In manchen Beispielen könnten auch mehrere Suchbild-Sequenz nacheinander erfasst werden. Ein solches Szenario ist in FIG. 3 im Zusammenhang mit Box 3011 beschrieben. Dabei wird zunächst in Box 3015 eine erste Suchbild-Sequenz, der eine erste Suchregion zugeordnet ist, aufgenommen; und dann in Box 3025 eine zweite Suchbild-Sequenz, der eine zweite Suchregion zugeordnet ist, aufgenommen. Dazwischen ist es in Box 3020 möglich, ein oder mehrere Suchbilder der ersten Suchbild-Sequenz aus Box 3015 auszuwerten und basierend auf diesem Auswerten Werte für mindestens einen Bildgebungsparameter des OP-Mikroskops, der zum Aufnehmen der zweiten Suchbild-Sequenz anschließenden Box 3025 verwendet wird, zu bestimmen.

Dieses Auswerten in Box 3020 könnte zum Beispiel das Bestimmen einer Anordnung eines ortsfest zum Patienten fixierten Markers in den ein oder mehreren Suchbildern der ersten Suchbild-Sequenz aus Box 3015 umfassen.

Derart könnte zum Beispiel die Anordnung der zweiten Suchregion, für welche die zweite Suchbild-Sequenz in Box 3025 erfasst wird, in Bezug auf die erste Suchregion der ersten Suchbild-Sequenz aus Box 3015 bestimmt werden. Es könnte auch ermittelt werden, ob ein Suchbild der ersten Suchbild-Sequenz für die zweite Suchbild-Sequenz wiederverwendet wird. Es könnte zum Beispiel ein Beleuchtungsparameter für das Aufnehmen der zweiten Suchbild-Sequenz eingestellt werden. Es könnte ein Vergrößerungsfaktor für die Suchbilder der zweiten Suchbild-Sequenz eingestellt werden.

Werden mehrere Suchbild-Sequenzen verwendet - wie beispielsweise in FIG. 3 im Zusammenhang mit Box 3015 und Box 3025 dargestellt - so ist es grundsätzlich möglich, dass zumindest ein Suchbild Teil mehrerer Suchbild-Sequenzen ist.

Nachfolgend wird in TAB. 1 ein Workflow beschrieben, welche das Verfahren der FIG. 3 implementieren können.

**Tab. 1: beispielhafte Implementierung eines Workflows, der die Bestimmung einer Transformationsvorschrift zwischen Bildern, die mittels eines OP-Mikroskops erfasst werden, und präoperativen Volumenbilddaten ermöglicht. Die Schritte müssen nicht zwangsläufig starr in dieser Reihenfolge ausgeführt werden, sondern können auch kombiniert werden bzw. parallel laufen um ggf. bereits aufgenommene Daten wiederzuverwenden.**

| Schritt | Kurze Beschreibung | Beispielhafte Details |
|---|---|---|
| 1 | Start Steuerungsskript | Zunächst startet der Anwender die Patientenregistrierung über einen Befehl, z.B. einen Knopfdruck, Fußschaltschult, Sprache, ... |
| | | Das bedeutet, dass ein entsprechendes Steuerungsskript, welches die nachfolgenden Schritte automatisch durchführt, gestartet wird. |
| 2 | Grob-Ausrichtung | Das OP-Mikroskop wird in einer Referenzanordnung positioniert, beispielsweise gerade nach unten auf einen Kopfbereich einer Liege zeigend. Dazu kann ein Motor entsprechend angesteuert werden. |
| | | Das robotische Operationsmikroskop wird automatisch so ausgerichtet, dass die Umfeldkamera in negative z-Richtung orientiert ist (nach unten zeigt) und der mögliche Arbeitsabstand maximiert wird. Alternativ oder zusätzlich könnte auch eine andere Kamera, z.B. eine Kamera einer Mikroskopie-Bildgebungseinheit, in negativer z-Richtung orientiert sein. |
| | | Dann ist das OP-Mikroskop "grob" über den Patienten gebracht, d.h. es kann angenommen werden, dass sich die Kameras des OP-Mikroskops räumlich betrachtet in z-Richtung (orthogonal vom Boden nach oben) oberhalb des Patienten befindet. |
| | | Vgl. FIG. 3: Box 3005. |
| 3 | Initialisierung | Dann wird - falls einstellbar - der Zoomfaktor der Umfeldkamera zuvor automatisch auf das Minimum eingestellt. Optional wird die Lichtquelle des Operationsmikroskop in ein Sensitivmodus geschaltet, um das Auge des Patienten (es wird angenommen, dass das Lid geschlossen ist) zu schützen. D.h. die Lichtintensität wird auf einen vergleichsweise niedrigen Wert eingestellt. |
| | | Der Zoomfaktor und die Helligkeit der Beleuchtung sind nur zwei Beispiele für Bildgebungsparameter, die auf einen vorgegebenen Wert gesetzt werden können, um eine Initialisierung zu erreichen. |
| | | Vgl. FIG. 3: Box 3010. |
| 4 | Erkennung Marker | Dann wird ungefähre Lage des Patientenkopfes bestimmt. Dazu werden Suchbilder einer Umfeldkamera (d.h. einer Übersichtsbild-Bildgebungseinheit) dahingehend ausgewertet, ob ein Marker, welcher am Patientenkopf befestigt ist oder ortsfest dazu fixiert ist (beispielsweise an einer Mayfield-Klemme bzw. einem Ring, der starr mit der Klemme verbunden ist), sichtbar ist. |
| | | Anstatt der Umfeldkamera könnte auch eine Mikroskopie-Bildgebungseinheit verwendet werden. Beispielsweise könnten stereoskopische Suchbilder erfasst werden. |
| | | Es wird also eine erste Suchbild-Sequenz aufgenommen. |
| | | Anschließend führt das Operationsmikroskop - unter gleichzeitiger Bildauswertung - so lange eine scannende Bewegung (z.B. spiralförmig nach außen) in |
| | | der xy Ebene durch, bis der Marker im Bild gefunden und seine Position relativ zur Trackingkamera bzw. allgemein in Bezug auf das OP-Mikroskop bestimmt werden konnte. Andere Scanmuster zum Abrastern einer Suchregion sind denkbar. Allgemein sind 2-D oder 3-D Scanmuster denkbar. Weitere Beispiele wären sphärische Muster oder Muster, die eine Pivot-Bewegung umfassen. |
| | | Optional wäre es denkbar, während des Aufnehmens der Suchbilder laufend zu erkennen, ob das Auge des Patienten geöffnet ist. Falls ja, wird der Scanvorgang automatisch unterbrochen/abgebrochen bzw. das Licht für den Bereich des Auges ausgeschaltet. |
| | | Vgl. FIG. 3: Box 3015, 3020. |
| | | Manchmal ist es denkbar, dass die relative Positionierung des OP-Mikroskops in Bezug auf einen Marker bereits bekannt ist, beispielsweise basierend auf Trackingdaten, die von einem Trackingsystem empfangen werden. Dann ist es nicht erforderlich, Suchbilder eine entsprechenden Suchbild-Sequenz zum Zwecke der Bestimmung der Anordnung des Markers zu erfassen, sondern die Anordnung des Markers kann basierend auf den Trackingdaten bestimmt werden. |
| 5 | Erkennung Patientenkopf als Zielregion | Mit dem Wissen, wo sich der Marker im Raum befindet wird nun die Position des Patientenkopfes in einem definierten Volumen um den Marker gesucht. Die entsprechende Suchregion ist also basierend auf der Anordnung des Markers im Raum bestimmt, sowie basierend auf Vorwissen betreffend die typische Kopfgröße in allen Raumrichtungen um den Marker bestimmt. Allgemein können Werte für mindestens einen Bildgebungsparameter für das Aufnehmen von |
| | | Suchbildern basierend auf Vorwissen betreffend einer Anordnung des Patienten in Bezug auf das OP-Mikroskop und/oder einer Anordnung der Zielregion in Bezug auf einen Marker bestimmt werden. |
| | | Optional kann durch die Kenntnis der Lagerung des Patienten und Anbringung des Markers (ob rechts, links, oben, unten etc.) die Suchregion für diese zweite Suchbild-Sequenz eingegrenzt werden. |
| | | Das OP-Mikroskop scannt die Suchregion ab (oder verwendet zumindest einen Teil der Suchbilder aus Schritt 4). |
| | | Eine Analyse-Modul erkennt den Patientenkopf als Zielregion. Dazu wird beispielsweise über Bildverarbeitung des Analyse-Moduls (z.B. Ansätze des Maschinellen Lernens) automatisch Hautgewebe bzw. bestimmte Anatomien (etwa Mund, Nase oder Augenhöhlen) im Bild erkannt. |
| | | Ergänzend oder alternativ werden auch Topographiedaten (d.h. Messdaten mit Tiefenauflösung) erstellt, so dass die dreidimensionalen Daten gegen eine typische Form des menschlichen Schädels gematcht werden können und so ein zusätzliches Indiz zur Erkennung des Patientenkopfes liefern. |
| | | Vgl. FIG. 3: Box 3025, 3030.. |
| 6 | Messdaten | Nachdem die Lage bzw. allgemein Anordnung des Patientenkopfes als Zielregion bekannt ist, wird die Topographie des Patientenkopfes erfasst. Dazu werden Messdaten aufgenommen, die eine Tiefenauflösung aufweisen. |
| | | Optional kann auch hier eine Rasterung erfolgen, d.h. eine automatische Bewegung des OP-Mikroskops zum Stitching des Sichtfelds verwendet werden. Es wäre denkbar, Suchbilder aus Schritt 4 oder 5 wiederverwendet, um die Scanzeit zu reduzieren. |
| | | Vgl. FIG. 3: Box 3035. |
| 7 | Filtern | Die Messdaten werden optional (automatisch) bereinigt, d.h. Gebiete mit Nicht-Hautgewebe (z.B. Tücher) werden entfernt und/oder auch metallische Halterungen / Instrumente werden entfernt. |
| | | Dies erfolgt entweder über die Auswertung eines Bildkontrasts (automatische Erkennung von Hautgewebe) und/oder über die topographischen Informationen (z.B. Strukturen, die nicht direkt mit dem Schädel verbunden sind und einen Abstand >5cm aufweisen, werden entfernt). |
| | | Vgl. FIG. 3: Box 3036. |
| 8 | Transformationsvorschrift | Die ermittelte Topographie wird für die Patientenregistrierung verwendet (beispielsweise an ein Navigationssystem gesendet. |
| | | Vgl. FIG. 3: Box 3040. |

Zusammenfassend wurden voranstehend Techniken beschrieben, welche eine automatische Erkennung eines zu scannenden Bereichs für die Patientenregistrierung, das heißt einer Zielregion, ermöglichen. Eine automatische Ausrichtung eines robotischen Visualisierungssystems, so dass Messdaten erfasst werden können, die eine Tiefenauflösung aufweisen und die Zielregion abbilden, wurde offenbart.

Selbstverständlich können die Merkmale der vorab beschriebenen Ausführungsformen und Aspekte der Erfindung miteinander im Rahmen der Ansprüche kombiniert werden.

## Patentansprüche

1. Computer-implementiertes Verfahren, das umfasst:
- Ansteuern eines robotischen Visualisierungssystems (801) zum Aufnehmen von mindestens einem Suchbild (111-113), das einen Patienten (805) zumindest teilweise abbildet, wobei das robotische Visualisierungssystem angesteuert wird, um mindestens eine Suchbilder-Sequenz aufzunehmen, wobei jede der mindestens einen Suchbilder-Sequenz mehrere entsprechende Suchbilder umfasst,
- während des Aufnehmens der mindestens einen Suchbilder-Sequenz, Ansteuern mindestens eines Motors des robotischen Visualisierungssystems, um das robotische Visualisierungssystem mehrfach umzupositionieren, sodass die mehreren Suchbilder der jeweiligen Suchbilder-Sequenz eine der jeweiligen Suchbilder-Sequenz zugeordnete Suchregion abrastern, wobei einer ersten Suchbilder-Sequenz der mindestens einen Suchbilder-Sequenz eine erste Suchregion zugeordnet ist, wobei einer zweiten Suchbilder-Sequenz der mindestens einen Suchbilder-Sequenz eine zweite Suchregion zugeordnet ist, wobei das robotische Visualisierungssystem zuerst zum Aufnehmen der ersten Suchbilder-Sequenz angesteuert wird und anschließend zum Aufnehmen der zweiten Suchbilder-Sequenz angesteuert wird,
- Auswerten von ein oder mehreren Suchbildern der ersten Suchbilder-Sequenz und basierend auf dem Auswerten der ein oder mehreren Suchbilder der ersten Suchbilder-Sequenz, Bestimmen von Werten für mindestens einen Bildgebungsparameter des robotischen Visualisierungssystems, der zum Aufnehmen der zweiten Suchbilder-Sequenz verwendet wird,
- Bestimmen einer Anordnung einer Zielregion (131) in dem mindestens einen Suchbild (111-113), und
- basierend auf der Anordnung der Zielregion (131) in dem mindestens einen Suchbild (111-113), Ansteuern des robotischen Visualisierungssystems (801) zum Aufnehmen von Messdaten mit Tiefenauflösung, wobei die Messdaten indikativ für eine Topographie einer Anatomie des Patienten (805) sind, um derart das Bestimmen einer Transformationsvorschrift zwischen mittels des robotischen Visualisierungssystems aufgenommenen Bildern und präoperativen Volumenbilddaten des Patienten zu ermöglichen.

2. Computer-implementiertes Verfahren nach Anspruch 1,
wobei das Auswerten der ein oder mehreren Suchbilder der ersten Suchbilder-Sequenz das Bestimmen einer Anordnung eines ortsfest zum Patienten fixierten Markers in den ein oder mehreren Suchbildern der ersten Suchbilder-Sequenz umfasst, und
wobei die Werte für den mindestens einen Bildgebungsparameter basierend auf der Anordnung des ortsfest zum Patienten fixierten Markers bestimmt werden.

3. Computer-implementiertes Verfahren nach Anspruch 1 oder 2,
wobei der mindestens eine Bildgebungsparameter eine Anordnung der zweiten Suchregion in Bezug auf die erste Suchregion umfasst.

4. Computer-implementiertes Verfahren nach einem der voranstehenden Ansprüche,
wobei der mindestens eine Bildgebungsparameter eine Wiederverwendung eines Suchbilds der ersten Suchbilder-Sequenz für die zweite Suchbilder-Sequenz umfasst.

5. Computer-implementiertes Verfahren nach einem der voranstehenden Ansprüche,
wobei der mindestens eine Bildgebungsparameter einen Beleuchtungsparameter für das Aufnehmen der zweiten Suchbilder-Sequenz umfasst.

6. Computer-implementiertes Verfahren nach einem der voranstehenden Ansprüche,
wobei zumindest ein Suchbild sowohl Teil der ersten Suchbild-Sequenz wie auch Teil der zweiten Suchbild-Sequenz ist.

7. Computer-implementiertes Verfahren nach einem der voranstehenden Ansprüche,
wobei das Verfahren weiterhin umfasst:
- Auswerten von ein oder mehreren Suchbildern der zweiten Suchbilder-Sequenz zum Erkennen von Hautgewebe des Patienten in den ein oder mehreren Suchbildern der zweiten Suchbilder-Sequenz,
wobei die Anordnung der Zielregion (131) basierend auf dem Erkennen des Hautgewebes bestimmt wird.

8. Computer-implementiertes Verfahren nach einem der voranstehenden Ansprüche, wobei das Verfahren weiterhin umfasst:
- Empfangen von Trackingdaten von einem zum robotischen Visualisierungssystem kollokiertem Trackingsystem, wobei die Trackingdaten eine Anordnung eines ortsfest zum Patienten fixierten Markers in Bezug auf das robotische Visualisierungssystem beschreiben, und
- Bestimmen von Werten für mindestens einen Bildgebungsparameter des robotischen Visualisierungssystems, der zum Aufnehmen der mindestens einen Suchbilder-Sequenz verwendet wird, basierend auf den Trackingdaten.

9. Computer-implementiertes Verfahren nach einem der voranstehenden Ansprüche, wobei das Verfahren weiterhin umfasst:
- Bestimmen von Werten für mindestens einen Bildgebungsparameter des robotischen Visualisierungssystems, der zum Aufnehmen der mindestens einen Suchbilder-Sequenz verwendet wird, basierend auf Vorwissen betreffend zumindest eines einer Anordnung des Patienten in Bezug auf das robotische Visualisierungssystems und einer Anordnung der Zielregion (131) in Bezug zu einem ortsfest zum Patienten fixierten Marker.

10. Computer-implementiertes Verfahren nach einem der voranstehenden Ansprüche, wobei das Verfahren weiterhin umfasst:
- während des Aufnehmens der mindestens einen Suchbilder-Sequenz, Auswerten von bereits erfassten Suchbildern der mindestens einen Suchbilder-Sequenz, und
- in Abhängigkeit von dem Auswerten, Abbrechen des Aufnehmens der mindestens einen Suchbilder-Sequenz und/oder Anpassen von Werten für mindestens einen Bildgebungsparameter des robotischen Visualisierungssystems, der zum Aufnehmen der mindestens einen Suchbilder-Sequenz verwendet wird.

11. Computer-implementiertes Verfahren nach einem der voranstehenden Ansprüche,
wobei die Messdaten mit einer Messmodalität erfasst werden, die aus folgender Gruppe ausgewählt ist: stereoskopische Bildgebung; Lichtlaufzeit-Messung; strukturierte Beleuchtung; und Defokus-Tiefenschätzung, und/oder
wobei die Messdaten zumindest eines des mindestens einen Suchbilds umfassen.

12. Computer-implementiertes Verfahren nach einem der voranstehenden Ansprüche, wobei das Verfahren weiterhin umfasst:
- Filtern (3036) der Messdaten zum Entfernen von Datenelementen, die nicht Hautgewebe des Patienten abbilden.

13. Computer-implementiertes Verfahren nach einem der voranstehenden Ansprüche, wobei das Verfahren weiterhin umfasst:
- Ansteuern (3005) eines Motors des robotischen Visualisierungssystems (801), um das robotische Visualisierungssystem (801) vor dem Aufnehmen des mindestens einen Suchbilds (111-113) in einer vorgegebenen Referenzanordnung anzuordnen, und/oder
- Setzen (3010) mindestens eines Bildgebungsparameters des robotischen Visualisierungssystems (801) vor dem Aufnehmen des mindestens einen Suchbilds (111-113) auf einen vorgegeben Wert.

14. Computer-implementiertes Verfahren nach einem der voranstehenden Ansprüche,
wobei das Verfahren basierend auf einem vordefinierten Steuerungsskript automatisiert durchgeführt wird,
wobei das Steuerungsskript ein Analyse-Modul zur Auswertung von Bilddaten oder Messdaten umfasst,
wobei das Steuerungsskript ein Positionierung-Modul zur Positionierung des robotischen Visualisierungssystems umfasst.

15. Computer-implementiertes Verfahren nach einem der voranstehenden Ansprüche, wobei das Verfahren weiterhin umfasst:
- Erzeugen eines Topographie-Datensatzes basierend auf den Messdaten,
- Durchführen einer Registrierung des Topographie-Datensatzes mit den präoperativen Volumenbilddaten, und
- basierend auf der Registrierung, Bestimmen der Transformationsvorschrift.

16. Datenverarbeitungseinheit (910), die eingerichtet ist, um die folgenden Schritte auszuführen:
- Ansteuern eines robotischen Visualisierungssystems zum Aufnehmen von mindestens einem Suchbild, das einen Patienten zumindest teilweise abbildet, wobei das robotische Visualisierungssystem angesteuert wird, um mindestens eine Suchbilder-Sequenz aufzunehmen, wobei jede der mindestens einen Suchbilder-Sequenz mehrere entsprechende Suchbilder umfasst,
- während des Aufnehmens der mindestens einen Suchbilder-Sequenz, Ansteuern mindestens eines Motors des robotischen Visualisierungssystems, um das robotische Visualisierungssystem mehrfach umzupositionieren, sodass die mehreren Suchbilder der jeweiligen Suchbilder-Sequenz eine der jeweiligen Suchbilder-Sequenz zugeordnete Suchregion abrastern, wobei einer ersten Suchbilder-Sequenz der mindestens einen Suchbilder-Sequenz eine erste Suchregion zugeordnet ist, wobei einer zweiten Suchbilder-Sequenz der mindestens einen Suchbilder-Sequenz eine zweite Suchregion zugeordnet ist, wobei das robotische Visualisierungssystem zuerst zum Aufnehmen der ersten Suchbilder-Sequenz angesteuert wird und anschließend zum Aufnehmen der zweiten Suchbilder-Sequenz angesteuert wird,
- Auswerten von ein oder mehreren Suchbildern der ersten Suchbilder-Sequenz und basierend auf dem Auswerten der ein oder mehreren Suchbilder der ersten Suchbilder-Sequenz, Bestimmen von Werten für mindestens einen Bildgebungsparameter des robotischen Visualisierungssystems, der zum Aufnehmen der zweiten Suchbilder-Sequenz verwendet wird,
- Bestimmen einer Anordnung einer Zielregion (131) in dem mindestens einen Suchbild, und
- basierend auf der Anordnung der Zielregion (131) in dem mindestens einen Suchbild, Ansteuern des robotischen Visualisierungssystems zum Aufnehmen von Messdaten mit Tiefenauflösung, wobei die Messdaten indikativ für eine Topographie einer Anatomie des Patienten sind, um derart das Bestimmen einer Transformationsvorschrift zwischen mittels des robotischen Visualisierungssystems aufgenommenen Bildern und präoperativen Volumenbilddaten des Patienten zu ermöglichen.

## Claims

1. Computer-implemented method, comprising:
- actuating a robotic visualization system (801) for recording at least one search image (111-113) which at least partly images a patient (805), wherein the robotic visualization system is actuated in order to record at least one search image sequence, wherein each of the at least one search image sequence comprises a plurality of corresponding search images,
- during the recording of the at least one search image sequence, actuating at least one motor of the robotic visualization system in order to reposition the robotic visualization system a number of times, such that the plurality of search images of the respective search image sequence scan a search region assigned to the respective search image sequence, wherein a first search region is assigned to a first search image sequence of the at least one search image sequence, wherein a second search region is assigned to a second search image sequence of the at least one search image sequence, wherein the robotic visualization system firstly is actuated for recording the first search image sequence and subsequently is actuated for recording the second search image sequence,
- evaluating one or more search images of the first search image sequence and, based on evaluating the one or more search images of the first search image sequence, determining values for at least one imaging parameter of the robotic visualization system which is used for recording the second search image sequence,
- determining an arrangement of a target region (131) in the at least one search image (111-113), and
- based on the arrangement of the target region (131) in the at least one search image (111-113), actuating the robotic visualization system (801) for recording measurement data with depth resolution, wherein the measurement data are indicative of a topography of an anatomy of the patient (805) in order in this way to enable the determination of a transformation specification between images recorded by means of the robotic visualization system and preoperative volume image data of the patient.

2. Computer-implemented method according to Claim 1,
wherein evaluating the one or more search images of the first search image sequence comprises determining an arrangement of a marker stationarily fixed with respect to the patient in the one or more search images of the first search image sequence, and
wherein the values for the at least one imaging parameter are determined based on the arrangement of the marker stationarily fixed with respect to the patient.

3. Computer-implemented method according to Claim 1 or 2,
wherein the at least one imaging parameter comprises an arrangement of the second search region in relation to the first search region.

4. Computer-implemented method according to any of the preceding claims,
wherein the at least one imaging parameter comprises a reuse of a search image of the first search image sequence for the second search image sequence.

5. Computer-implemented method according to any of the preceding claims,
wherein the at least one imaging parameter comprises an illumination parameter for recording the second search image sequence.

6. Computer-implemented method according to any of the preceding claims,
wherein at least one search image is both part of the first search image sequence and part of the second search image sequence.

7. Computer-implemented method according to any of the preceding claims,
wherein the method furthermore comprises:
- evaluating one or more search images of the second search image sequence for recognizing skin tissue of the patient in the one or more search images of the second search image sequence,
wherein the arrangement of the target region (131) is determined based on recognizing the skin tissue.

8. Computer-implemented method according to any of the preceding claims, wherein the method furthermore comprises:
- receiving tracking data from a tracking system collocated with respect to the robotic visualization system, wherein the tracking data describe an arrangement of a marker stationarily fixed with respect to the patient in relation to the robotic visualization system, and
- determining values for at least one imaging parameter of the robotic visualization system which is used for recording the at least one search image sequence, based on the tracking data.

9. Computer-implemented method according to any of the preceding claims, wherein the method furthermore comprises:
- determining values for at least one imaging parameter of the robotic visualization system which is used for recording the at least one search image sequence, based on prior knowledge concerning at least one out of an arrangement of the patient in relation to the robotic visualization system and an arrangement of the target region (131) in relation to a marker stationarily fixed with respect to the patient.

10. Computer-implemented method according to any of the preceding claims, wherein the method furthermore comprises:
- during the recording of the at least one search image sequence, evaluating already captured search images of the at least one search image sequence, and
- depending on the evaluating, terminating the recording of the at least one search image sequence and/or adapting values for at least one imaging parameter of the robotic visualization system which is used for recording the at least one search image sequence.

11. Computer-implemented method according to any of the preceding claims,
wherein the measurement data are captured with a measurement modality selected from the following group: stereoscopic imaging; time-of-flight measurement; structured illumination; and defocus depth estimation, and/or
wherein the measurement data comprise at least one of the at least one search image.

12. Computer-implemented method according to any of the preceding claims, wherein the method furthermore comprises:
- filtering (3036) the measurement data in order to remove data elements which do not image skin tissue of the patient.

13. Computer-implemented method according to any of the preceding claims, wherein the method furthermore comprises:
- actuating (3005) a motor of the robotic visualization system (801) in order to arrange the robotic visualization system (801) in a predefined reference arrangement before the recording of the at least one search image (111-113), and/or
- setting (3010) at least one imaging parameter of the robotic visualization system (801) to a predefined value before the recording of the at least one search image (111-113).

14. Computer-implemented method according to any of the preceding claims,
wherein the method is carried out in an automated manner based on a predefined control script,
wherein the control script comprises an analysis module for evaluating image data or measurement data,
wherein the control script comprises a positioning module for positioning the robotic visualization system.

15. Computer-implemented method according to any of the preceding claims, wherein the method furthermore comprises:
- generating a topography data set based on the measurement data,
- carrying out a registration of the topography data set with the preoperative volume image data, and
- based on the registration, determining the transformation specification.

16. Data processing unit (910), configured to carry out the following steps:
- actuating a robotic visualization system for recording at least one search image which at least partly images a patient, wherein the robotic visualization system is actuated in order to record at least one search image sequence, wherein each of the at least one search image sequence comprises a plurality of corresponding search images,
- during the recording of the at least one search image sequence, actuating at least one motor of the robotic visualization system in order to reposition the robotic visualization system a number of times, such that the plurality of search images of the respective search image sequence scan a search region assigned to the respective search image sequence, wherein a first search region is assigned to a first search image sequence of the at least one search image sequence, wherein a second search region is assigned to a second search image sequence of the at least one search image sequence, wherein the robotic visualization system firstly is actuated for recording the first search image sequence and subsequently is actuated for recording the second search image sequence,
- evaluating one or more search images of the first search image sequence and, based on evaluating the one or more search images of the first search image sequence, determining values for at least one imaging parameter of the robotic visualization system which is used for recording the second search image sequence,
- determining an arrangement of a target region (131) in the at least one search image, and
- based on the arrangement of the target region (131) in the at least one search image, actuating the robotic visualization system for recording measurement data with depth resolution, wherein the measurement data are indicative of a topography of an anatomy of the patient in order in this way to enable the determination of a transformation specification between images recorded by means of the robotic visualization system and preoperative volume image data of the patient.

## Revendications

1. Procédé mis en œuvre par ordinateur, qui comprend les étapes consistant à :
- commander un système de visualisation robotique (801) pour acquérir au moins une image de recherche (111-113) qui représente un patient (805) au moins partiellement, le système de visualisation robotique étant commandé pour acquérir au moins une séquence d'images de recherche, chacune de la ou des séquences d'images de recherche comprenant de multiples images de recherche correspondantes,
- pendant l'acquisition de ladite au moins une séquence d'images de recherche, commander au moins un moteur du système de visualisation robotique pour repositionner le système de visualisation robotique plusieurs fois, de sorte que les multiples images de recherche de la séquence d'images de recherche respective balayent une région de recherche associée à la séquence d'images de recherche respective, une première région de recherche étant associée à une première séquence d'images de recherche de ladite au moins une séquence d'images de recherche, une deuxième région de recherche étant associée à une deuxième séquence d'images de recherche de ladite au moins une séquence d'images de recherche, le système de visualisation robotique étant d'abord commandé pour acquérir la première séquence d'images de recherche puis commandé pour acquérir la deuxième séquence d'images de recherche,
- évaluer une ou plusieurs images de recherche de la première séquence d'images de recherche et, sur la base de l'évaluation de ladite ou desdites images de recherche de la première séquence d'images de recherche, déterminer des valeurs pour au moins un paramètre d'imagerie du système de visualisation robotique qui est utilisé pour acquérir la deuxième séquence d'images de recherche,
- déterminer une disposition d'une région cible (131) dans ladite au moins une image de recherche (111-113), et
- sur la base de la disposition de la région cible (131) dans ladite au moins une image de recherche (111-113), commander le système de visualisation robotique (801) pour acquérir des données de mesure avec une résolution de profondeur, les données de mesure étant indicatives d'une topographie d'une anatomie du patient (805), de manière à permettre ainsi la détermination d'une règle de transformation entre des images acquises au moyen du système de visualisation robotique et des données d'image de volume de pré-chirurgie du patient.

2. Procédé mis en œuvre par ordinateur selon la revendication 1,
dans lequel l'évaluation de ladite ou desdites images de recherche de la première séquence d'images de recherche comprend la détermination d'une disposition d'un marqueur fixé de manière stationnaire par rapport au patient dans ladite ou lesdites images de recherche de la première séquence d'images de recherche, et
dans lequel les valeurs pour ledit au moins un paramètre d'imagerie sont déterminées sur la base de la disposition du marqueur fixé de manière stationnaire par rapport au patient.

3. Procédé mis en œuvre par ordinateur selon la revendication 1 ou 2,
dans lequel ledit au moins un paramètre d'imagerie comprend une disposition de la deuxième région de recherche par rapport à la première région de recherche.

4. Procédé mis en œuvre par ordinateur selon l'une quelconque des revendications précédentes,
dans lequel ledit au moins un paramètre d'imagerie comprend une réutilisation d'une image de recherche de la première séquence d'images de recherche pour la deuxième séquence d'images de recherche.

5. Procédé mis en œuvre par ordinateur selon l'une quelconque des revendications précédentes,
dans lequel ledit au moins un paramètre d'imagerie comprend un paramètre d'éclairage pour acquérir la deuxième séquence d'images de recherche.

6. Procédé mis en œuvre par ordinateur selon l'une quelconque des revendications précédentes,
dans lequel au moins une image de recherche fait partie à la fois de la première séquence d'images de recherche et de la deuxième séquence d'images de recherche.

7. Procédé mis en œuvre par ordinateur selon l'une quelconque des revendications précédentes,
dans lequel le procédé comprend en outre les étapes consistant à :
- évaluer une ou plusieurs images de recherche de la deuxième séquence d'images de recherche pour reconnaître le tissu cutané du patient dans ladite ou lesdites images de recherche de la deuxième séquence d'images de recherche,
dans lequel la disposition de la région cible (131) est déterminée sur la base de la reconnaissance du tissu cutané.

8. Procédé mis en œuvre par ordinateur selon l'une quelconque des revendications précédentes, dans lequel le procédé comprend en outre les étapes consistant à :
- recevoir des données de suivi d'un système de suivi colocalisé avec le système de visualisation robotique, les données de suivi décrivant une disposition d'un marqueur fixé de manière stationnaire au patient par rapport au système de visualisation robotique, et
- déterminer des valeurs pour au moins un paramètre d'imagerie du système de visualisation robotique, qui est utilisé pour acquérir ladite au moins une séquence d'images de recherche, sur la base des données de suivi.

9. Procédé mis en œuvre par ordinateur selon l'une quelconque des revendications précédentes, dans lequel le procédé comprend en outre les étapes consistant à :
- déterminer des valeurs pour au moins un paramètre d'imagerie du système de visualisation robotique, qui est utilisé pour acquérir ladite au moins une séquence d'images de recherche, sur la base de connaissances préalables concernant au moins l'une d'une disposition du patient par rapport au système de visualisation robotique et d'une disposition de la région cible (131) par rapport à un marqueur fixé de manière stationnaire par rapport au patient.

10. Procédé mis en œuvre par ordinateur selon l'une quelconque des revendications précédentes, dans lequel le procédé comprend en outre les étapes consistant à :
- pendant l'acquisition de ladite au moins une séquence d'images de recherche, évaluer des images de recherche déjà acquises de ladite au moins une séquence d'images de recherche, et
- en fonction de l'évaluation, interrompre l'acquisition de ladite au moins une séquence d'images de recherche et/ou ajuster des valeurs pour au moins un paramètre d'imagerie du système de visualisation robotique qui est utilisé pour l'acquisition de ladite au moins une séquence d'images de recherche.

11. Procédé mis en œuvre par ordinateur selon l'une quelconque des revendications précédentes,
dans lequel les données de mesure sont acquises selon une modalité de mesure qui est choisie dans le groupe suivant : imagerie stéréoscopique ; mesure du temps de propagation de la lumière ; éclairage structuré ; et estimation de profondeur par défocalisation, et/ou dans lequel les données de mesure comprennent au moins l'une de la ou des images de recherche.

12. Procédé mis en œuvre par ordinateur selon l'une quelconque des revendications précédentes, dans lequel le procédé comprend en outre les étapes consistant à :
- filtrer (3036) les données de mesure pour retirer des éléments de données qui ne représentent pas le tissu cutané du patient.

13. Procédé mis en œuvre par ordinateur selon l'une quelconque des revendications précédentes, dans lequel le procédé comprend en outre les étapes consistant à :
- commander (3005) un moteur du système de visualisation robotique (801) pour positionner le système de visualisation robotique (801) dans une disposition de référence prédéterminée avant d'acquérir ladite au moins une image de recherche (111-113), et/ou
- régler (3010) au moins un paramètre d'imagerie du système de visualisation robotique (801) à une valeur prédéterminée avant d'acquérir ladite au moins une image de recherche (111-113).

14. Procédé mis en œuvre par ordinateur selon l'une quelconque des revendications précédentes,
le procédé étant exécuté de manière automatisée sur la base d'un script de commande prédéfini,
le script de commande comprenant un module d'analyse destiné à évaluer des données d'image ou des données de mesure,
le script de commande comprenant un module de positionnement destiné à positionner le système de visualisation robotique.

15. Procédé mis en œuvre par ordinateur selon l'une quelconque des revendications précédentes, dans lequel le procédé comprend en outre les étapes consistant à :
- générer un ensemble de données de topographie sur la base des données de mesure,
- effectuer un recalage de l'ensemble de données de topographie avec les données d'image de volume de pré-chirurgie, et
- déterminer la règle de transformation sur la base du recalage.

16. Unité de traitement de données (910), qui est configurée pour exécuter les étapes consistant à :
- commander un système de visualisation robotique pour acquérir au moins une image de recherche qui représente un patient au moins partiellement, le système de visualisation robotique étant commandé pour acquérir au moins une séquence d'images de recherche, chacune de la ou des séquences d'images de recherche comprenant de multiples images de recherche correspondantes,
- pendant l'acquisition de ladite au moins une séquence d'images de recherche, commander au moins un moteur du système de visualisation robotique pour repositionner le système de visualisation robotique plusieurs fois, de sorte que les multiples images de recherche de la séquence d'images de recherche respective balayent une région de recherche associée à la séquence d'images de recherche respective, une première région de recherche étant associée à une première séquence d'images de recherche de ladite au moins une séquence d'images de recherche, une deuxième région de recherche étant associée à une deuxième séquence d'images de recherche de ladite au moins une séquence d'images de recherche, le système de visualisation robotique étant d'abord commandé pour acquérir la première séquence d'images de recherche puis commandé pour acquérir la deuxième séquence d'images de recherche,
- évaluer une ou plusieurs images de recherche de la première séquence d'images de recherche et, sur la base de l'évaluation de ladite ou desdites images de recherche de la première séquence d'images de recherche, déterminer des valeurs pour au moins un paramètre d'imagerie du système de visualisation robotique qui est utilisé pour acquérir la deuxième séquence d'images de recherche,
- déterminer une disposition d'une région cible (131) dans ladite au moins une image de recherche, et
- sur la base de la disposition de la région cible (131) dans ladite au moins une image de recherche, commander le système de visualisation robotique pour acquérir des données de mesure avec une résolution de profondeur, les données de mesure étant indicatives d'une topographie d'une anatomie du patient, de manière à permettre ainsi la détermination d'une règle de transformation entre des images acquises au moyen du système de visualisation robotique et des données d'image de volume de pré-chirurgie du patient.
